# EUROPEAN PATENT SPECIFICATION

(11) **EP 2 189 100 B1**
(45) Date of publication and mention of the grant of the patent: **24.05.2017**
(21) Application number: 08739316.1
(22) Date of filing: 28.03.2008
(51) Int. Cl.: A61B 1/04, A61B 17/00, A61B 1/00, A61M 5/142, A61B 34/00, A61B 90/00, A61M 5/14

(54) **MEDICAL APPARATUS**
MEDIZINISCHES GERÄT
APPAREIL MÉDICAL

(30) Priority: 20.09.2007 JP 2007244206
(43) Date of publication of application: 26.05.2010
(73) Proprietor: Olympus Corporation, Tokyo 192-8507 (JP)
(72) Inventor: ASADA, Daisuke, Tokyo 151-0072 (JP); KARASAWA, Hitoshi, Tokyo 151-0072 (JP); HANDA, Keiji, Tokyo 151-0072 (JP); NAKAJIMA, Sho, Tokyo 151-0072 (JP)
(74) Representative: Gunzelmann, Rainer
(86) International application number: PCT/JP2008/056198
(87) International publication number: WO 2009/037880

(56) References cited:
- WO-A1-2005/122866
- WO-A1-2007/077922
- WO-A1-2007/078003
- WO-A1-2007/078003
- WO-A1-2007/097393
- JP-A- 8 089 504
- JP-A- 8 089 504
- JP-A- 8 336 497
- JP-A- 2005 323 681
- JP-A- 2007 260 397
- US-A1- 2003 114 731
- US-A1- 2005 165 449
- US-A1- 2007 073 102
- US-A1- 2007 161 855

## Description

### Technical Field

The present invention relates to a medical apparatus that includes a medical instrument that is fixed inside the body, and more particularly to a medical apparatus that makes the orientation of the medical instrument movable from outside the body.

### Background Art

It is known that an endoscope that is a medical instrument includes an image pickup apparatus, and is introduced into a body cavity of a patient to perform various examinations and treatments of a diseased part inside a body by means of an observation image that is photographed by the image pickup apparatus.

Such endoscopes include an endoscope that is introduced from an oral cavity or the anus into a digestive organ such as the esophagus, the stomach, the colon, or the duodenum that are luminal tracts inside the body, and an endoscope that is introduced into an abdominal cavity from the vicinity of the navel region by puncturing and penetrating a body wall. Generally, the endoscope has a long insertion portion, and the insertion portion is inserted into the digestive tract or into an abdominal cavity.

Recently a capsule-type medical apparatus as described, for example, in Japanese Patent Application Laid-Open Publication No. 2005-237979 has been proposed for the purpose of alleviating patient pain that is caused by introducing the insertion portion. The aforementioned Japanese Patent Application Laid-Open Publication No. 2005-237979 discloses technology for a capsule-type endoscope apparatus that is capable of reaching a target region inside a lumen while rotating upon reception of a rotating magnetic field from outside the body.

However, although the capsule-type endoscope apparatus disclosed in Japanese Patent Application Laid-Open Publication No. 2005-237979 is effective technology for inside a luminal tract, it would be difficult to divert use of this conventional capsule-type endoscope apparatus technology to endoscopic observation inside an abdominal cavity.

That is, as a conventional method of introducing an endoscope into an abdominal cavity, for example, a surgical operation, namely, laparoscopic surgery, is performed that introduces an endoscope into an abdominal cavity without creating a large opening in the abdomen to thereby ensure minimal invasion. In this operation, the abdomen of a patient is punctured with a trocar for guiding an endoscope for observation into a body cavity and a trocar for guiding a treatment instrument to a treatment site, and curative treatment is performed while observing the treatment instrument and the treatment site with the endoscope. According to this method, although a treatment site can be observed in detail using the endoscope, there is the problem that the range of the observable field of view is relatively narrow. Therefore, in addition to the normal endoscope, it is preferable to use an image pickup apparatus such as an endoscope for wide-angle observation in which a wide-angle field of view range is set such that an entire treatment site in an abdominal cavity can be extensively observed.

However, when the laparoscopic surgery is performed using, in addition to the normal endoscope, an endoscope for wide-angle observation within an abdominal cavity by puncturing an abdominal wall that is a body wall with a trocar, it is necessary to puncture the abdominal wall of a patient with a plurality of trocars. In that case, there is the problem that inserting a plurality of trocars imposes a larger burden on a patient than heretofore, and the laparoscopic surgery is no longer minimally invasive.

Further, with regard to an image pickup apparatus that can extensively observe an entire area in an abdominal cavity, when the image pickup apparatus is in a state in which, with respect to a region that is photographed, a treatment site is positioned at the center of the image, and is fixed in a state of observing in a direction that is approximately the same as the vertical and horizontal directions of an observation image of a normal endoscope, the surgeon does not experience a sense of incongruity with respect to the images.
Therefore, it is preferable that the desired observation direction of an image pickup apparatus that is introduced into an abdominal cavity separately from a normal endoscope can be adjusted.Document US2007073102 A1 discloses an endoscope apparatus that is introduced into a body cavity. The apparatus comprises an internal insertion portion and an extra-corporeal operation unit. The internal insertion portion comprises a base portion for fixing the endoscope apparatus to a body wall inside the body cavity, a motor connected to the endoscope apparatus such that a posture of the endoscope can be changed. The motor is connected to the base portion in a rotatable manner. The internal insertion portion further comprises an intermediate body which is interposed between the endoscope and and the base portion and movably connects the endoscope to the base portion. The extra-corporeal operation unit is installed on a body surface outside the body and has magnets in the operation unit to synchronously move the internal insertion portion to an optional position inside of the body cavity, by magnetic force.

Document US 2005/0165449 A1 discloses a device and a system for manipulating a surgical tool and an intended location including a surgical anchor having at least one opening, wherein the opening provides a catch for a pin, and at least one anchor point to position and orient a surgical tool inside a human body. In one embodiment, the surgical anchor may be rotated by means of a dual external magnetic stack. Document JP 8-089504 concerns an intracoelom ultrasonic probe with which operability of the top end hard part of an ultrasonic probe inside a coelom and adhesion with its internal organs can be improved.

The present invention has been made in view of the aforementioned problems, and it is an object of the present invention to provide a medical apparatus that is capable of performing a minimally invasive surgical operation without increasing a burden on a patient, and that can also freely change the orientation of a medical instrument that has been fixedly installed inside a body to a desired direction.

### Disclosure of Invention

### Means for Solving the Problem

To achieve the above object, a medical apparatus of the present invention includes the features of claim 1. Preferred embodiments are defined in the dependent claims.

### Brief Description of the Drawings

Fig. 1 is a view illustrating a configuration of an endoscope system that is a medical apparatus according to a first embodiment of the present invention;
Fig. 2 is a sectional view illustrating a configuration of an extracorporeal device according to the first embodiment;
Fig. 3 is a top view illustrating the configuration of the extracorporeal device according to the first embodiment;
Fig. 4 is a sectional view illustrating a configuration of an intra-abdominal camera according to the first embodiment;
Fig. 5 is a sectional view along a line V-V in Fig. 4 according to the first embodiment;
Fig. 6 is a view illustrating a state in which an abdominal wall of a patient is punctured with trocars according to the first embodiment;
Fig. 7 is a sectional view illustrating a state in which a puncture needle is inserted into the extracorporeal device according to the first embodiment;
Fig. 8 is a view for explaining procedures for introducing an intra-abdominal camera into an abdominal cavity according to the first embodiment;
Fig. 9 is a view for explaining procedures for introducing an intra-abdominal camera into an abdominal cavity, that illustrates a state in which an abdominal wall is punctured with a puncture needle to hook a wire of the intra-abdominal camera according to the first embodiment;
Fig. 10 is a view for explaining procedures for fixing the intra-abdominal camera to an abdominal wall, that illustrates a state in which a puncture needle which has hooked a wire of the intra-abdominal camera is pulled up according to the first embodiment;
Fig. 11 is a view for explaining procedures for fixing an intra-abdominal camera to an abdominal wall, that illustrates a state in which a puncture needle is pulled up and the extracorporeal device is lowered along the puncture needle according to the first embodiment;
Fig. 12 is a sectional view of the extracorporeal device in the state shown in Fig. 11 according to the first embodiment;
Fig. 13 is a view illustrating a state in which the extracorporeal device is installed on an abdomen, and the intra-abdominal camera is fixed to the abdominal wall according to the first embodiment;
Fig. 14 is a sectional view of the extracorporeal device and the intra-abdominal camera in the state shown in FIG. 13 according to the first embodiment;
Fig. 15 is an overall configuration diagram of the endoscope system illustrating a state in which the intra-abdominal camera is fixed to the abdominal wall according to the first embodiment;
Fig. 16 is a sectional view for explaining an action whereby the intra-abdominal camera is rotatably movable about an axis thereof by means of an operation of the extracorporeal device according to the first embodiment;
Fig. 17 is a sectional view for explaining an action whereby the intra-abdominal camera is rotatably movable while disposed at an angle relative to the axis thereof by means of an operation of the extracorporeal device according to the first embodiment;
Fig. 18 is a sectional view for explaining procedures for extracting the extracorporeal device from a wire of the intra-abdominal camera according to the first embodiment;
Fig. 19 is a view for explaining procedures for taking the intra-abdominal camera out from the abdominal cavity according to the first embodiment;
Fig. 20 is a sectional view illustrating a configuration of a pharmaceutical spraying apparatus of a medical instrument according to a second embodiment of the present invention;
Fig. 21 is an overall configuration diagram of an endoscope system illustrating a state in which the pharmaceutical spraying apparatus is fixed to an abdominal wall according to the second embodiment; and
Fig. 22 is an overall configuration diagram of an endoscope system illustrating a modification example that shows a state in which an image pickup apparatus capable of magnified observation is fixed to an abdominal wall.

### Best Mode for Carrying Out the Invention

Hereunder, embodiments of the present invention will be described with reference to the drawings. In the following description, a medical apparatus that performs laparoscopic surgery will be described as an example.

### (First Embodiment)

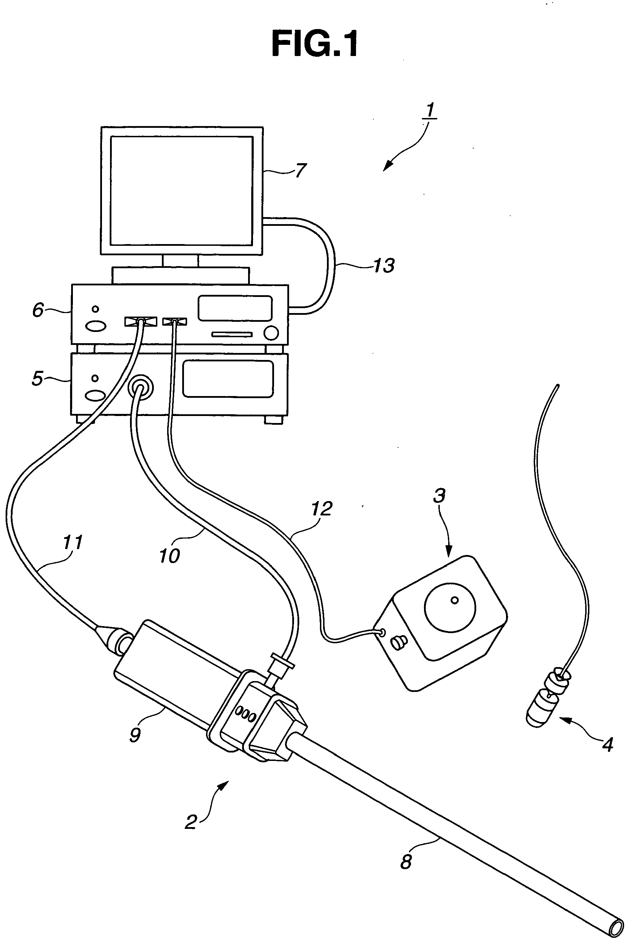

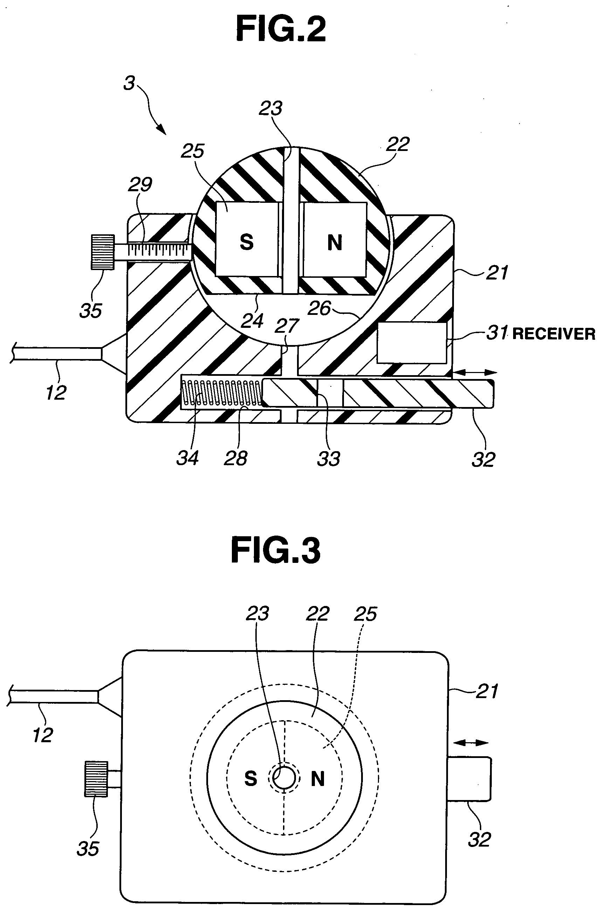

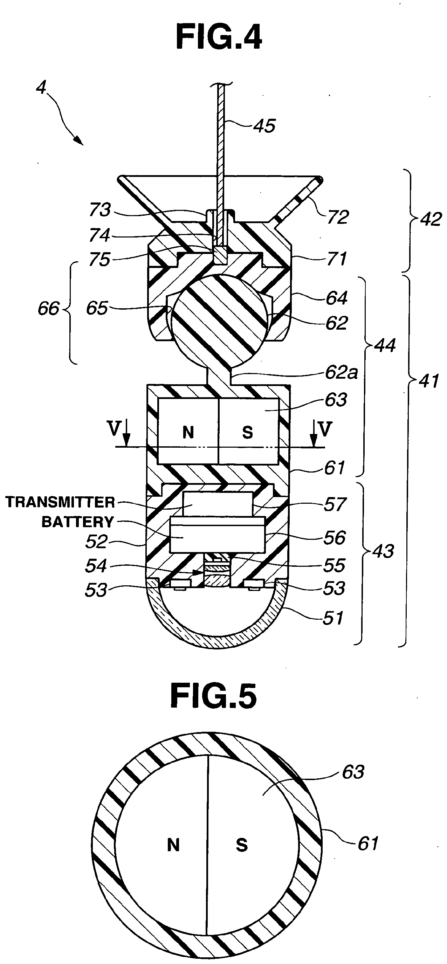

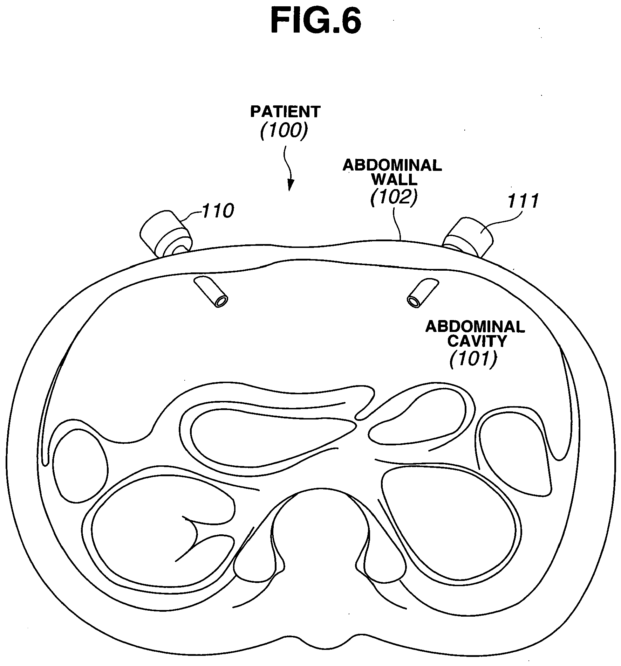

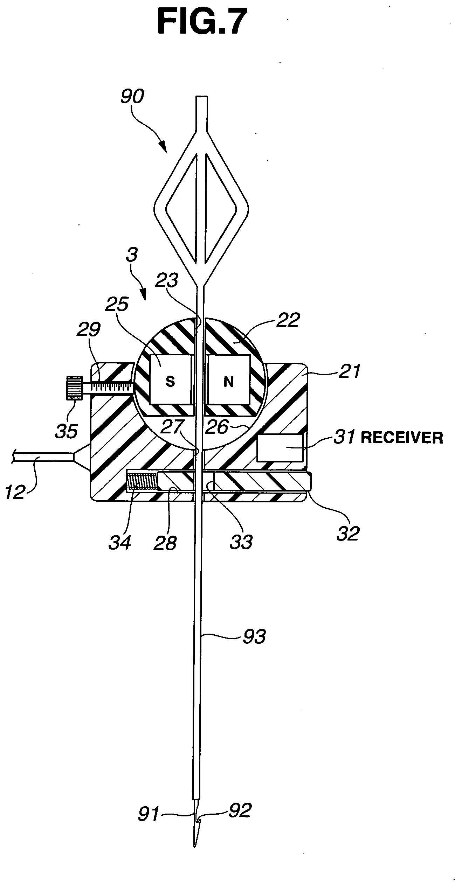

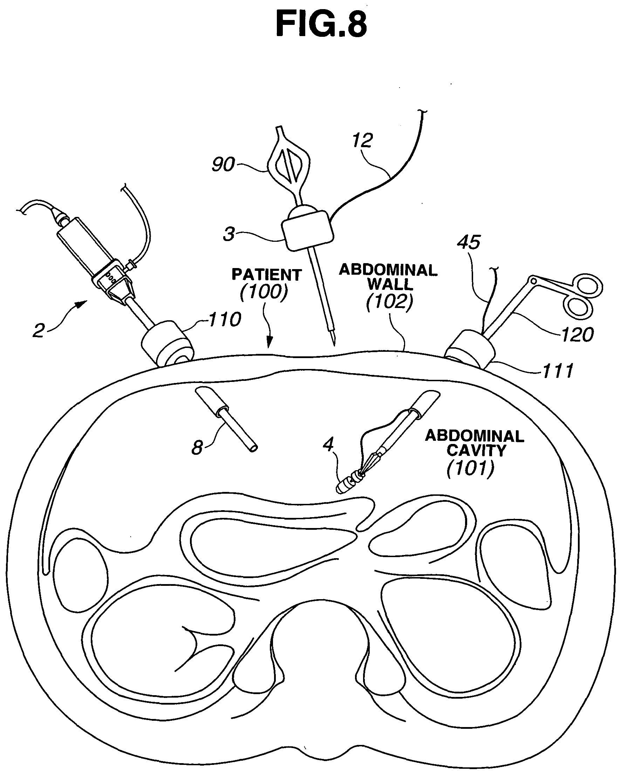

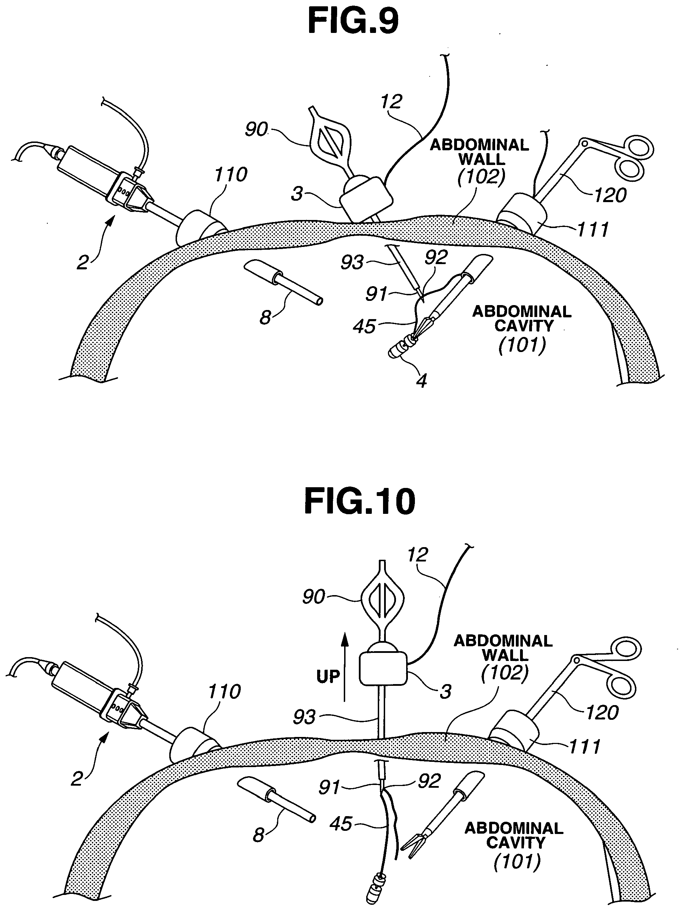

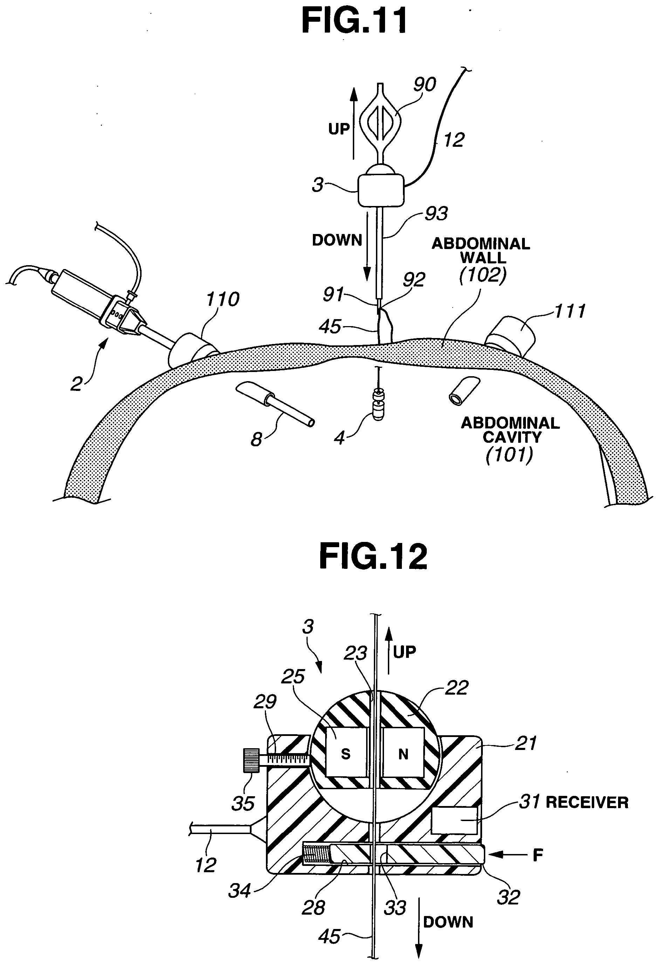

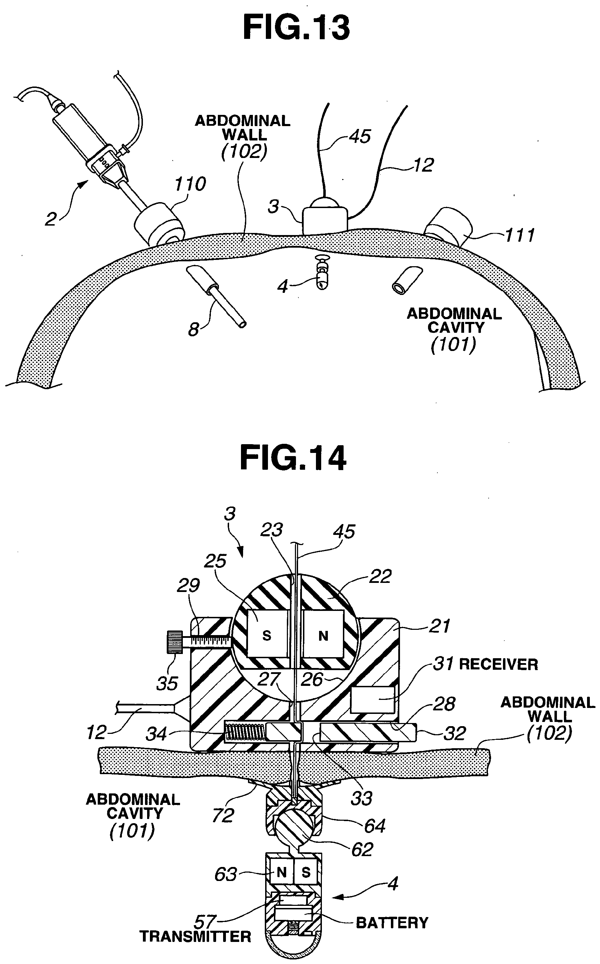

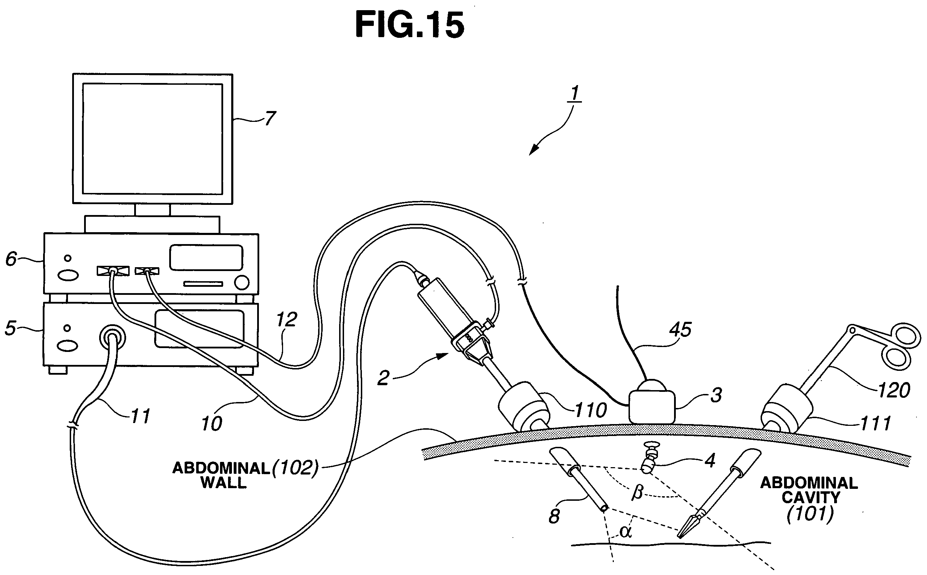

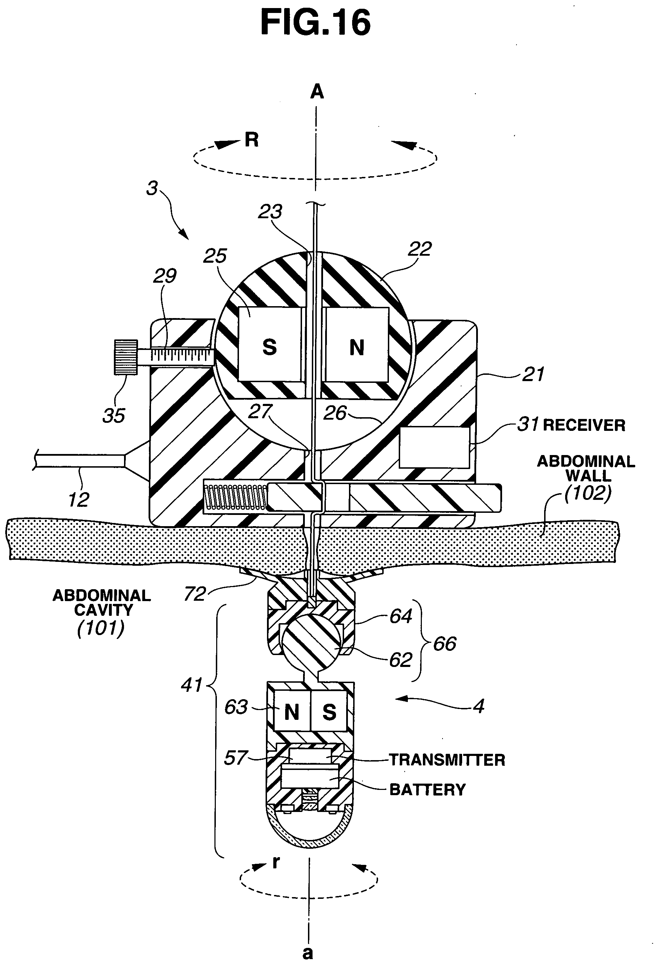

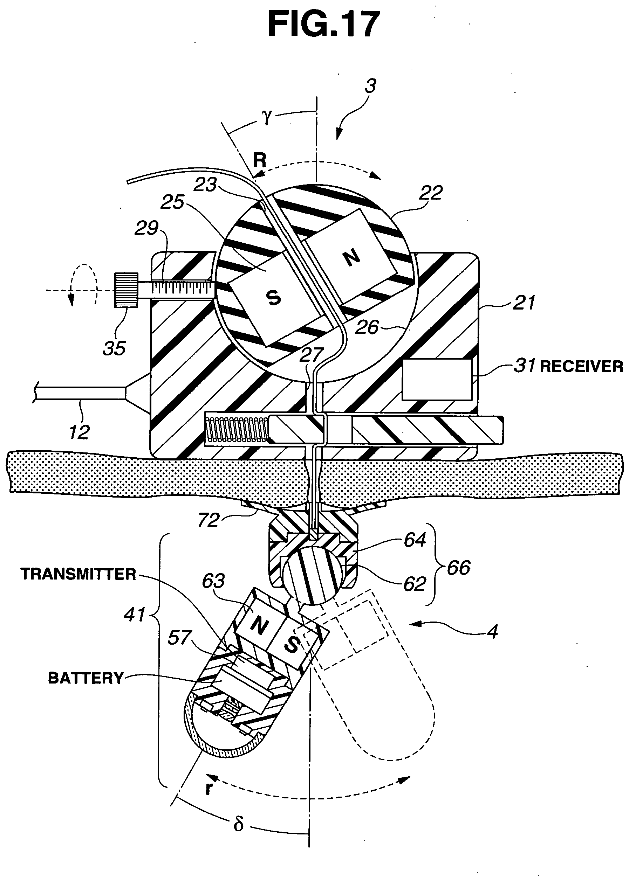

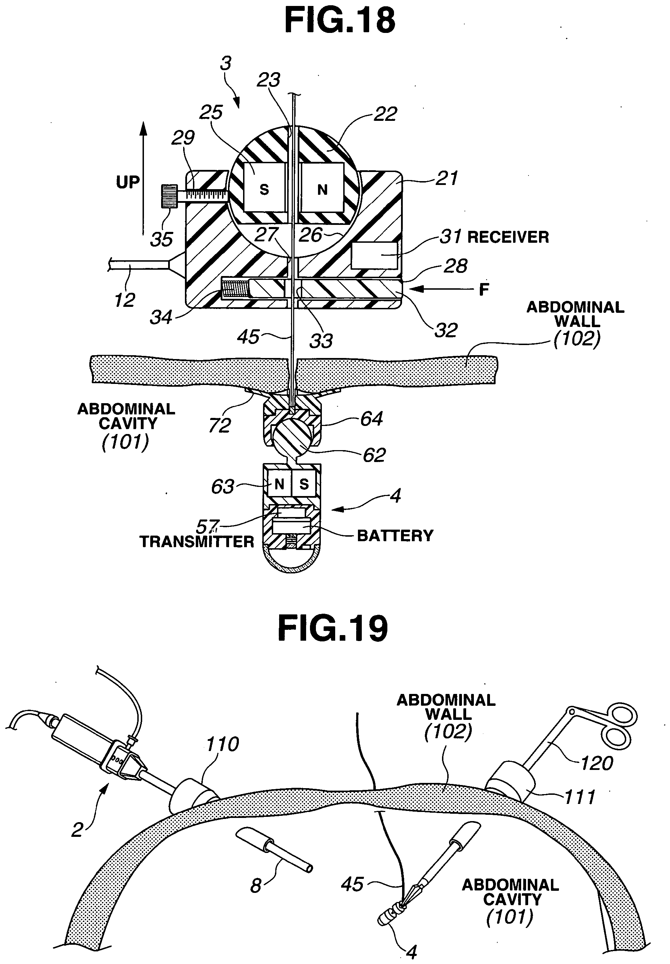

First, an endoscope system that is the medical apparatus according to the present invention that is used for laparoscopic surgery will be described. Fig. 1 to Fig. 19 relate to a first embodiment of the present invention. Fig. 1 is a view illustrating a configuration of an endoscope system that is a medical apparatus. Fig. 2 is a sectional view illustrating a configuration of an extracorporeal device. Fig. 3 is a top view illustrating the configuration of the extracorporeal device. Fig. 4 is a sectional view illustrating a configuration of an intra-abdominal camera. Fig. 5 is a sectional view along a line V-V in Fig. 4. Fig. 6 is a view illustrating a state in which an abdominal wall of a patient is punctured with trocars. Fig. 7 is a sectional view illustrating a state in which a puncture needle is inserted into the extracorporeal device. Fig. 8 is a view for explaining procedures for introducing an intra-abdominal camera into an abdominal cavity. Fig. 9 is a view for explaining procedures for introducing an intra-abdominal camera into an abdominal cavity, that illustrates a state in which an abdominal wall is punctured with a puncture needle to hook a wire of the intra-abdominal camera. Fig. 10 is a view for explaining procedures for fixing the intra-abdominal camera to an abdominal wall, that illustrates a state in which a puncture needle which has hooked a wire of the intra-abdominal camera is pulled up. Fig. 11 is a view for explaining procedures for fixing an intra-abdominal camera to an abdominal wall, that illustrates a state in which a puncture needle is pulled up and the extracorporeal device is lowered along the puncture needle. Fig. 12 is a sectional view of the extracorporeal device in the state shown in Fig. 11. Fig. 13 is a view illustrating a state in which the extracorporeal device is installed on an abdomen, and the intra-abdominal camera is fixed to the abdominal wall. Fig. 14 is a sectional view of the extracorporeal device and the intra-abdominal camera in the state shown in FIG. 13. Fig. 15 is an overall configuration diagram of an endoscope system illustrating a state in which an intra-abdominal camera is fixed to an abdominal wall. Fig. 16 is a sectional view for explaining an action whereby the intra-abdominal camera is rotatably movable about an axis thereof by means of an operation of the extracorporeal device. Fig. 17 is a sectional view for explaining an action whereby the intra-abdominal camera is rotatably movable while disposed at an angle relative to the axis thereof by means of an operation of the extracorporeal device. Fig. 18 is a sectional view for explaining procedures for extracting the extracorporeal device from a wire of the intra-abdominal camera. Fig. 19 is a view for explaining procedures for taking the intra-abdominal camera out from the abdominal cavity.

As shown in Fig. 1, an endoscope system 1 of the present embodiment that performs laparoscopic surgery is mainly constituted by a rigid endoscope 2 as a first photographing apparatus, an extracorporeal device 3 as an extracorporeal-side posture control apparatus, an extremely small intra-abdominal camera (hereunder, abbreviated to "camera") 4 as a second photographing apparatus and also an image pickup apparatus, a light source 5, a camera control unit (hereunder, abbreviated to "CCU") 6 as a signal processing device with a built-in image processing circuit, and a display device 7 that is connected to the CCU 6 by a communication cable 13 and displays an observation image.

The light source 5 supplies an illuminating light to an illuminating optical system provided in the rigid endoscope 2. The light source 5 and the rigid endoscope 2 are detachably connected by a light source cable 10.

The rigid endoscope 2 is mainly constituted by a rigid insertion portion 8, and an operation portion 9 sequentially connected to a proximal end of the insertion portion 8. An image guide and a light guide bundle are inserted through the inside of the insertion portion 8 of the rigid endoscope 2. The insertion portion 8 is also provided with, on a distal end surface thereof, a photographing optical system for condensing a subject image onto a rigid endoscope camera, described later, via the image guide, and the illuminating optical system for irradiating an illuminating light from the light guide bundle toward a subject.

An unshown camera head in which a solid-state image pickup device such as a CCD or a CMOS is disposed is built into the operation portion 9 of the rigid endoscope 2. An optical image of an observation site illuminated by the illuminating light supplied from the light source 5 to the rigid endoscope 2 through the light source cable 10 is picked up by the camera head in the operation portion 9 through the image guide in the insertion portion 8. The rigid endoscope camera photoelectrically converts the picked-up optical image into an image pickup signal. The image pickup signal is transmitted to the CCU 6 through an image pickup cable 11. In the rigid endoscope 2 of the present embodiment, an image pickup optical system is set such that an angle of view α (see Fig. 15) available for photographing is, for example, 70° to 75°.

The CCU 6 generates a video signal from the transmitted image signal, and outputs the video signal to the display device 7. The display device 7 is, for example, a liquid crystal display. The display device 7 receives the video signal outputted from the CCU 6, and displays both a normal observation image picked up with the rigid endoscope 2 and a wide-angle observation image picked up with the camera 4 on one screen or switches the normal observation image and the wide-angle observation image to separately display the images on the screen. The CCU 6 is removably connected to the extracorporeal device 3 by an electric cable 12.

Next, the extracorporeal device 3 will be described in detail below with reference to Figs. 2 and 3.

As shown in Figs. 2 and 3, the extracorporeal device 3 has a receiver 31 inside a housing 21. An extracorporeal-side posture adjustment portion 22 that is an extracorporeal posture control portion is rotatably installed in the housing 21 that is formed of a non-magnetic material. The extracorporeal-side posture adjustment portion 22 has a spherical shape in which a part thereof has been severed.

The extracorporeal-side posture adjustment portion 22 has a spherical body formed of a synthetic resin that is a non-magnetic material such as plastic, with a hole portion 23 passing through the spherical body at the center and with a part (a lower part, in this case) of the spherical body severed to form a flat portion 24. The extracorporeal-side posture adjustment portion 22 has an extracorporeal-side permanent magnet 25 disposed therein around the hole portion 23, the extracorporeal-side permanent magnet 25 being a cylindrical extracorporeal-side ferromagnetic member. The extracorporeal-side permanent magnet 25 has a north pole and a south pole that are magnetically separated by a plane along the hole portion 23.

The extracorporeal-side posture adjustment portion 22 is movably disposed in a spherical concave portion 26 which has a similar spherical shape and opens at top of the housing 21. That is, the extracorporeal device 3 has a so-called trackball mechanism that makes the posture adjustment portion 22 rotatably movable with respect to the housing 21.

The housing 21 has a wire passage hole 27 that communicates with a center lower part of the spherical concave portion 26 and is located on an extension of a center line of the extracorporeal-side posture adjustment portion 22 so as to open to an underside of the housing 21. Further, in the housing 21, a wire fixing lever 32 (described later) that communicates with the wire passage hole 27 is slidably formed in a lateral direction, and a slide hole portion 28 is formed that opens on one side surface (in this case, the right side surface). Furthermore, a screw hole 29 is formed in the housing 21 that opens on the other side surface (in this case, the left side surface). The screw hole 29 communicates with the spherical concave portion 26, and a posture position fixing screw 35, described later, is screwed into the screw hole 29.

The wire fixing lever 32 that is formed of non-magnetic material and which has an urging spring 34 fixed to an end surface thereof is inserted into and disposed in the slide hole portion 28 of the housing 21. The wire fixing lever 32 has a substantially rectangular parallelepiped shape, and a hole portion 33 is formed therein which communicates with the wire passage hole 27 of the housing 21 by sliding the wire fixing lever 32 in the inward direction of the housing 21.

The posture position fixing screw 35 is made of non-magnetic material and screwed into the screw hole 29 of the housing 21 to serve as a posture fixing portion. When the posture position fixing screw 35 is screwed deeply enough into the screw hole 29, the extracorporeal-side posture adjustment portion 22 abuts against an inner end face of the housing 21, thereby restraining movement of the extracorporeal-side posture adjustment portion 22 in the spherical concave portion 26.

Next, the camera 4 will be described in detail below with reference to Figs. 4 and 5.

The camera 4 is mainly constituted by a camera body 41 and an abdominal wall fixing portion 42 which are sequentially provided, as shown in Fig. 4 and Fig. 5.

The camera body 41 includes a so-called capsule-type image pickup unit 43 and an intracorporeal-side posture adjustment portion 44 that is a driven posture control portion.

The outer shape of the image pickup unit 43 is formed with a substantially dome-shaped transparent hood 51 on a distal end side (lower side in Fig. 4), and a camera housing 52 formed of non-magnetic material in which the transparent hood 51 is disposed so as to hermetically seal one surface.

The camera housing 52 is provided with a plurality of (in this case, two) white LEDs 53 that are illuminating portions which are disposed as light sources of illuminating light on one surface on the transparent hood 51 side. The camera housing 52 is also provided with an objective lens group 54 held in a lens holding hole formed at substantially the center of the aforementioned surface, and a solid-state image pickup device unit 55 such as a CCD or a C-MOS in which a light-receiving portion is disposed at a position where a photographing light is condensed by the objective lens group 54.

A transmitter 57 is disposed inside the camera housing 52. A battery 56 that supplies power to the transmitter 57, the white LEDs 53, and the solid-state image pickup device unit 55 is also contained inside the camera housing 52. In a functional portion of the camera body 41 according to the present embodiment, an image pickup optical system that picks up an image over a wide-angle visual field area is set such that an angle of view β (see Fig. 15) available for photographing is 90° or more. An image signal that is photoelectrically converted by the solid-state image pickup device unit 55 is transmitted by radio transmission from the transmitter 57 to the receiver 31 of the extracorporeal device 3.

The intracorporeal-side posture adjustment portion 44 includes a main body portion 61 formed of non-magnetic material that is substantially cylindrical column in outer shape and is fitted into a proximal end (upper end in Fig. 4) of the camera housing 52; a sphere portion 62 formed integrally with an extending end of a neck portion 62a which, being made of the same material as the main body portion 61, extends from a center of the proximal end face of the main body portion 61; and a sphere receiving portion 64 formed of non-magnetic material that rotatably supports the sphere portion 62.

An intracorporeal-side permanent magnet 63 which is a cylindrical, intracorporeal-side ferromagnetic member is contained inside the main body portion 61. As shown in Fig. 5, the intracorporeal-side permanent magnet 63 has north and south poles that are magnetically separated by a plane along the center of the main body portion 61.

A concave portion 65 that houses and rotatably holds the sphere portion 62 is formed in the sphere receiving portion 64. This provides a ball joint portion 66 that constitutes a movable portion in which the sphere portion 62 is rotatably held inside the sphere receiving portion 64.

The abdominal wall fixing portion 42 is formed of, for example, a flexible elastic member such as silicone rubber. The abdominal wall fixing portion 42 includes a connecting portion 71 that is fitted to a proximal end portion of the sphere receiving portion 64, and a suction cup 72 at a rear end portion of the connecting portion 71. Further, in the abdominal wall fixing portion 42 is formed a convex portion 73 that projects in a cylindrical shape at substantially the center of a surface of the suction cup 72, and a through hole 74 that is formed in the center of the connecting portion 71 so as to communicate with a hole portion of the convex portion 73.

A hoisting wire 45 having a predetermined length is inserted through the through hole 74 of the abdominal wall fixing portion 42. A coupling portion 75 connected by caulking is provided at one end portion of the wire 45. The coupling portion 75 is fitted and fixed to the center of a proximal end surface of the sphere receiving portion 64. That is, the wire 45 is provided so as to extend from the center of the suction cup 72.

The endoscope system 1 of the present embodiment having the configuration described above is used for laparoscopic surgery and for treatment inside an abdominal cavity that is one of the body cavities of a patient.

Next, procedures for installing the camera 4 of the endoscope system 1 of the present embodiment in an abdominal cavity as a body cavity of a patient for laparoscopic surgery, and the operation thereof will be described in detail with reference to Figs. 6 to 19.

First, a surgeon makes two small dissections in an abdominal wall 102 of a patient 100 by using a surgical knife or the like, and punctures the dissections with trocars 110 and 111 as shown in Fig. 6. In this case, the surgeon makes a puncture into an abdominal cavity 101 with the trocar 111 for introducing a treatment instrument 120 such as a grasping forceps into the abdominal cavity 101 by dissecting the abdominal wall 102 or the like at another position that is separated by a predetermined distance from the trocar 110 for introducing the rigid endoscope 2 into the abdominal cavity 101.

Further, as shown in Fig. 7, the surgeon inserts an insertion portion 93 of a puncture needle 90 into the hole portion 23 provided in the extracorporeal-side posture adjustment portion 22 of the extracorporeal device 3. At this time, the surgeon pushes the wire fixing lever 32 into the housing 21 such that the puncture needle 90 penetrates the extracorporeal device 3, and inserts the puncture needle 90 such that the insertion portion 93 of the puncture needle 90 penetrates the hole portion 33 of the wire fixing lever 32.

The surgeon causes the insertion portion 93 to sufficiently project from a bottom surface of the extracorporeal device 3 in a manner such that the extracorporeal device 3 is located to an adequate degree on the side of the puncture needle 90 on which the hands of the surgeon are located (upper side in Fig. 7). In this state, a wall surface of the hole portion 33 of the wire fixing lever 32 is in abutment with and restrains the insertion portion 93 of the puncture needle 90 by means of an urging force of the urging spring 34 on the wire fixing lever 32 so that the extracorporeal device 3 does not fall off from the puncture needle 90. The puncture needle 90 has a hook portion 92 that is formed by notching in the needle portion 91.

Next, as shown in Fig. 8, the surgeon inserts the insertion portion 8 of the rigid endoscope 2 into the abdominal cavity 101 through the trocar 110. Subsequently, the surgeon inserts the camera 4 grasped by the treatment instrument 120 such as a grasping forceps into the abdominal cavity 101 through the trocar 111. At this time, the surgeon may insert the camera 4 into the abdominal cavity 101 while checking an image obtained by the rigid endoscope 2.

When the camera 4 is introduced into the abdominal cavity 101 through the trocar 111, the convex portion 73 that projects in a cylindrical shape from approximately the center of the surface of the suction cup 72 is grasped by the treatment instrument such as a grasping forceps. Since the convex portion 73 is provided at the approximate center of the adhering surface of the suction cup 72, the camera 4 can be easily grasped by the treatment instrument in a balanced manner. Consequently, the surgeon can easily pass the camera 4 through the trocar 111 when introducing the camera 4 into the abdominal cavity. That is, the surgeon can easily introduce the camera 4 into the abdominal cavity 101 without causing the camera 4 to get caught in the trocar 111.

Next, as shown in Fig. 9, while checking the image obtained by the rigid endoscope 2, the surgeon punctures the abdominal wall 102 with the puncture needle 90 which holds the extracorporeal device 3. Subsequently, the surgeon hooks the hook portion 92 formed in the needle portion 91 of the puncture needle 90 onto the wire 45 of the camera 4 while viewing the image obtained by the rigid endoscope 2.

Thereafter, as shown in Fig. 10, the surgeon extracts the puncture needle 90 to outside the body from the abdominal cavity 101 (in the upward direction in Fig. 10) in a state in which the wire 45 is hooked in the hook portion 92 of the needle portion 91. Subsequently, as shown in Fig. 11, along with extracting the puncture needle 90 from the abdominal cavity 101, the surgeon moves the extracorporeal device 3 relative to the insertion portion 93 of the puncture needle 90 in the direction of the abdomen (downward direction in Fig. 11) of the patient 100, and pulls the puncture needle 90 until the wire 45 passes through the hole portion 23 provided in the extracorporeal-side posture adjustment portion 22.

At this time, by pushing the wire fixing lever 32 of the extracorporeal device 3 towards the inside of the housing 21, the surgeon can easily slide the extracorporeal device 3 relative to the insertion portion 93 of the puncture needle 90. As shown in Fig. 12, when the wire 45 passes through the hole portion 23 of the extracorporeal-side posture adjustment portion 22, the surgeon moves the extracorporeal device 3 relative to the wire 45 in the direction of the abdomen of the patient 100 (downward direction in Fig. 12) while pulling the wire 45 itself (upward direction in Fig. 12).

That is, the surgeon can easily slide the extracorporeal device 3 relative to the insertion portion 93 of the puncture needle 90 and the wire 45 of the camera 4 by maintaining a state in which the wire fixing lever 32 of the extracorporeal device 3 is pushed towards the inside of the housing 21 (F direction in Fig. 12).

Subsequently, as shown in Fig. 13, the surgeon pulls the wire 45 of the camera 4 until the abdominal wall 102 is sandwiched between the extracorporeal device 3 and the camera 4 in a state in which the extracorporeal device 3 is placed on the abdomen of the patient 100. At this time, after confirming based on the image obtained by the rigid endoscope 2 that the suction cup 72 of the camera 4 is placed in intimate contact with an inner surface of the abdominal wall 102 as shown in Fig. 14, the surgeon stops pushing in the wire fixing lever 32 of the extracorporeal device 3.

The wire fixing lever 32 of the extracorporeal device 3 is thereby moved upon reception of the urging force of the urging spring 34, so that the hole portion 33 enters a state in which the hole portion 33 is misaligned with the wire passage hole 27 of the housing 21. The wire 45 inserted through the hole portion 33 and the wire passage hole 27 is caught therein and is thereby fixed to the housing 21. As a result, the extracorporeal device 3 and the camera 4 are fixed in a state in which the abdominal wall 102 is sandwiched therebetween.

Thus, as shown in Fig. 15, the camera 4 is installed in a reliably stable state in the abdominal cavity 101 of the patient 100, and laparoscopic surgery is performed by means of the endoscope system 1 of the present embodiment. In this connection, for example, one end portion of an unshown insufflation tube is attached to the trocar 110, and a carbon dioxide gas or the like is injected into the abdominal cavity as an insufflation gas for the purpose of securing a visual field of the rigid endoscope 2 and a region in which an operation instrument or the like is operated. As shown in Fig. 15, the surgeon inserts the rigid endoscope 2 through the trocar 110 and the treatment instrument 120 through the trocar 111 to perform laparoscopic surgery in a state in which the camera 4 is stuck to and retained at the abdominal wall 102 inside the abdominal cavity 101.

Next, the operations of the extracorporeal device 3 and the camera 4 of the endoscope system 1 of the present embodiment will be described in detail using Fig. 16 and Fig. 17.

As shown in Fig. 16, when the camera 4 is rotated around an axis A (R direction in Fig. 16) that is parallel to the hole portion 23 through which the extracorporeal-side posture adjustment portion 22 of the extracorporeal device 3 passes at the center thereof, the intracorporeal-side permanent magnet 63 that receives the magnetic force of the extracorporeal-side permanent magnet 25 follows the rotation and rotationally moves around a longitudinal axis a (r direction in Fig. 16).

More specifically, the intracorporeal-side permanent magnet 63 is constantly receiving a magnetic force that attracts the south pole of the intracorporeal-side permanent magnet 63 towards the north pole of the extracorporeal-side permanent magnet 25 and attracts the north pole of the intracorporeal-side permanent magnet 63 towards the south pole of the extracorporeal-side permanent magnet 25. Therefore, the camera body 41 of the camera 4 follows the rotation around the axis A of the extracorporeal-side posture adjustment portion 22, and rotates using the center of the sphere portion 62 of the ball joint portion 66 as a fulcrum.

This allows the surgeon to rotate the camera body 41 by operating the extracorporeal-side posture adjustment portion 22 of the extracorporeal device 3, and thus the surgeon can rotate an image picked up by the image pickup unit 43 for display on the display device 7 and thereby vertically or horizontally adjust a display position within the abdominal cavity. That is, by operating the extracorporeal-side posture adjustment portion 22 of the extracorporeal device 3, the surgeon can change left, right, top, and bottom positions of an image photographed by the camera 4 according to left, right, top, and bottom positions of an image photographed by the rigid endoscope 2, in a contactless manner using magnetic force. Accordingly, the surgeon can match the vertical and horizontal directions of two displayed images that are photographed by the rigid endoscope 2 and the camera 4, and thereby avoid feeling a sense of incongruity when viewing the images on the display device 7.

As shown in Fig. 17, when the extracorporeal-side posture adjustment portion 22 of the extracorporeal device 3 is rotated by a predetermined angle γ in the horizontal direction (R direction in Fig. 17), the intracorporeal-side permanent magnet 63 of the camera 4 receives the magnetic force of the extracorporeal-side permanent magnet 25 and is attracted thereby such that the intracorporeal-side permanent magnet 63 tilts at a predetermined angle δ in the horizontal direction (r direction in Fig. 17). The predetermined angles γ and δ depend on the mass of the camera body 41 of the camera 4, the intensities of the magnetic forces of the extracorporeal-side permanent magnet 25 and the intracorporeal-side permanent magnet 63, and the like.

That is, when the south pole of the extracorporeal-side permanent magnet 25 is brought close to the intracorporeal-side permanent magnet 63 by rotation, the magnetic force attracting the intracorporeal-side permanent magnet 63 to the north pole side increases. At this time, since the north pole of the extracorporeal-side permanent magnet 25 goes away from the intracorporeal-side permanent magnet 63 due to the rotation, the magnetic force that attracts the intracorporeal-side permanent magnet 63 to the south pole side decreases. Consequently, the camera body 41 of the camera 4 tracks the predetermined rotational angle γ in the horizontal direction of the extracorporeal-side posture adjustment portion 22, and thus tilts by the predetermined angle δ with the center of the sphere portion 62 of the ball joint portion 66 serving as a fulcrum.

Accordingly, since the surgeon can tilt the camera body 41 in a contactless manner using the magnetic force by operating the extracorporeal-side posture adjustment portion 22 of the extracorporeal device 3, the surgeon can adjust a display position so as to place an affected part to be treated at approximately the center of an image that is picked up by the image pickup unit 43 for display on the display device 7 or at a position that facilitates treatment of the affected part. That is, the surgeon can change a photographing direction of the camera 4 inside the abdominal cavity 101.

The surgeon can fix the camera 4 in a desired observation direction by screwing the posture position fixing screw 35 into the housing 21 to thereby fix the extracorporeal-side posture adjustment portion 22.

Subsequently, when the surgeon ends the laparoscopic surgery, as shown in Fig. 18, the surgeon extracts the extracorporeal device 3 from the wire 45 while pushing the wire fixing lever 32 of the extracorporeal device 3 to the inner side of the housing 21. Thereafter, as shown in Fig. 19, the surgeon grasps the camera 4 inside the abdominal cavity 101 with the treatment instrument 120 such as a grasping forceps and takes out the camera 4 to outside the body from the abdominal cavity 101 through the trocar 111.

The endoscope system 1 according to each of the embodiments as described above allows a surgeon to observe body tissue in a body cavity (in this case, the abdominal cavity 101) from multiple viewpoints including a wide-angle viewpoint. Thus, for example, the surgeon can easily recognize an entire resection line during surgery of a large organ or resection of the large intestine. Further, the endoscope system 1 allows the surgeon to easily adjust a visual field direction of the camera 4 that is introduced into the abdominal cavity 101 separately from the rigid endoscope 2 for magnified observation, as well as fix the visual field direction. Consequently, use of the endoscope system 1 according to the present invention makes it easy to administer treatment by laparoscopic surgery.

Main components of the extracorporeal device 3, including the housing 21, the extracorporeal-side posture adjustment portion 22, and the wire fixing lever 32, but excluding the extracorporeal-side permanent magnet 25 are made of non-magnetic material. Further, components of the camera 4, including the abdominal wall fixing portion 42 and the intracorporeal-side posture adjustment portion 44, but excluding the intracorporeal-side permanent magnet 63, are made of non-magnetic material. That is, the components disposed between the extracorporeal-side permanent magnet 25 of the extracorporeal device 3 and the intracorporeal-side permanent magnet 63 of the camera 4 are made of non-magnetic material. Thus, the camera 4 is constituted so as not to affect the magnetism of the permanent magnets 25 and 63 that are used by the extracorporeal device 3 for posture adjustment operations.

### (Second Embodiment)

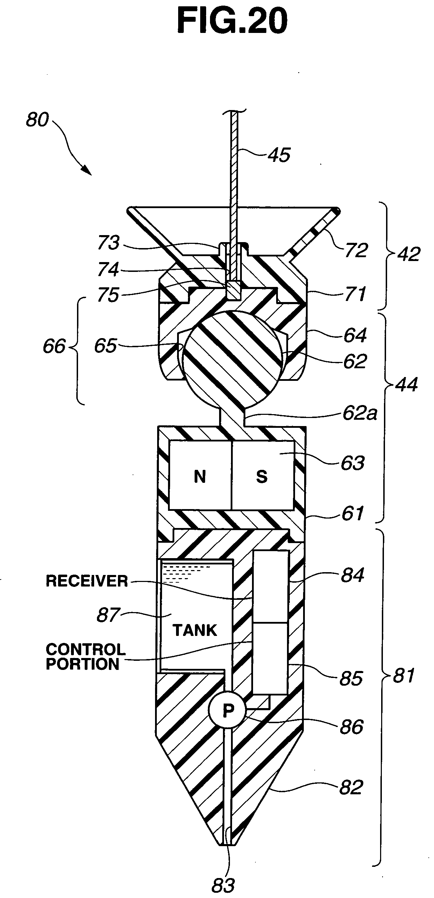

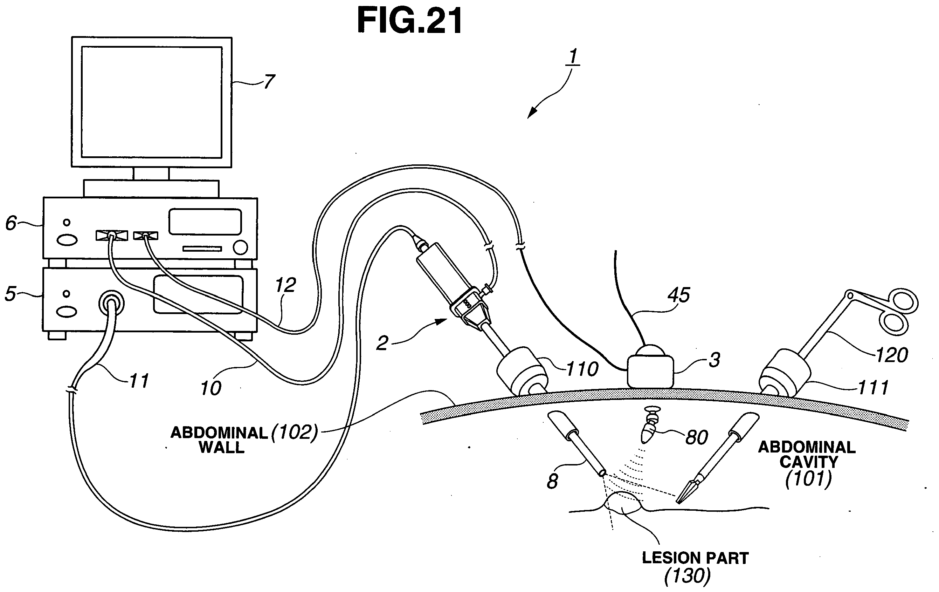

Next, a second embodiment according to the endoscope system of the present invention is described using Fig. 20 and Fig. 21. Fig. 20 and Fig. 21 relate to the second embodiment of the present invention. Fig. 20 is a view that illustrates a configuration of a pharmaceutical spraying apparatus. Fig. 21 is an overall configuration diagram of an endoscope system illustrating a state in which a pharmaceutical spraying apparatus is fixed to an abdominal wall. In the following description, the same reference numerals are used to denote components that are the same as in the endoscope system 1 of the first embodiment described above, and a detailed description of those components is omitted.

According to the present embodiment, an example is described in which the medical instrument to be installed inside the abdominal cavity 101 is changed from the intra-abdominal camera 4 of the first embodiment to a pharmaceutical spraying apparatus 80 that includes a functional portion that sprays a tumor specific pharmaceutical on tissue inside the body.

As shown in Fig. 20, the pharmaceutical spraying apparatus 80 of the present embodiment is provided with a pharmaceutical spraying apparatus main body portion 81 together with the intracorporeal-side posture adjustment portion 44. The pharmaceutical spraying apparatus main body portion 81 includes a housing 82 having a tapered nozzle shape and, inside the housing 82, a receiver 84, a control portion 85 which receives and is driven by a signal from the receiver 84, a micropump 86 that is drivingly controlled by the control portion 85 and is installed along a pharmaceutical solution spray path 83, and a tank 87 in which a pharmaceutical solution is stored. The tank 87 is a cartridge-type tank that is removable from the housing 82. The receiver 84, the control portion 85, and the micropump 86 are supplied with power by an unshown battery.

Similarly to the first embodiment, as shown in Fig. 21, the pharmaceutical spraying apparatus 80 configured in this manner is introduced inside the abdominal cavity 101 and fixed to the abdominal wall 102. The pharmaceutical spraying direction of the pharmaceutical spraying apparatus 80 can be changed to a desired direction by the extracorporeal device 3 to thereby spray a tumor specific pharmaceutical inside the tank 87 towards a lesion part 130.

That is, the extracorporeal device 3 is provided with an unshown transmitter, and an instruction signal from the transmitter is transmitted by radio communication to the receiver 84 of the pharmaceutical spraying apparatus 80. The receiver 84 outputs the received instruction signal to the control portion 85, and the control portion 85 drivingly controls the micropump 86.

Thus, the endoscope system 1 of the present embodiment is configured to allow operations to change the spraying direction of a pharmaceutical that is sprayed with the pharmaceutical spraying apparatus 80 inside the abdominal cavity 101 in a contactless manner using the extracorporeal device 3 that is outside the body.

Although not shown in the drawings, a configuration may also be adopted in which an aiming mechanism (such as a laser pointer) for enhancing the spraying accuracy by enabling confirmation of the spraying direction is incorporated into the pharmaceutical spraying apparatus 80.

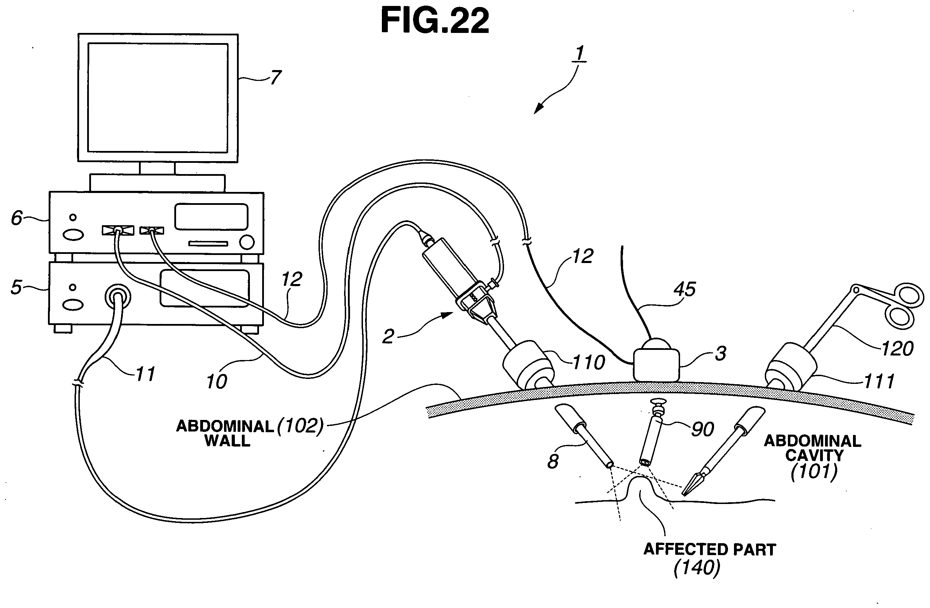

In the respective embodiments described above, examples have been described in which the medical instrument to be fixedly installed on the abdominal wall 102 inside the abdominal cavity 101 is the intra-abdominal camera 4 or the pharmaceutical spraying apparatus 80. However, as shown in Fig. 22, for example, the medical instrument may be an image pickup apparatus 95 that is capable of magnified observation of an affected part 140 inside the abdominal cavity 101. Fig. 22 is an overall configuration diagram of an endoscope system illustrating a state in which an image pickup apparatus that includes a functional portion capable of magnified observation is fixed to an abdominal wall.

Further, in the above embodiments, examples were described in which sending and receiving of various signals to the functional portion of various medical instruments (the intra-abdominal camera 4, the pharmaceutical spraying apparatus 80, and the image pickup apparatus 95 and the like) inside the abdominal cavity 101 and the extracorporeal device 3 are performed using radio communication by means of a receiver and a transmitter. However, a configuration may be adopted in which the wire 45 of each kind of medical instruments is changed to a transmission cable, and the transmission cable is directly connected to the CCU 6.

Further, the CCU 6 that processes images of the rigid endoscope 2, the intra-abdominal camera 4, the image pickup apparatus 95 and the like, as well as the display device 7 are not limited to a single device, and a configuration may be adopted that is provided with a plurality of the CCU 6 and the display device 7, respectively, in accordance with the number of medical instruments for observation to be used.

A mechanism that can change a visual field direction or spraying direction or the like of the various medical instruments (the intra-abdominal camera 4, the pharmaceutical spraying apparatus 80, the image pickup apparatus 95 and the like) installed inside the abdominal cavity 101 by operation of the extracorporeal device 3 according to the above described embodiments is not limited to the configuration of the permanent magnets 25 and 63 described above. For example, a magnetic field generating device disclosed in Japanese Patent Application Laid-Open Publication No. 2007-215583 that is known technology may be used.

The magnetic field generating device disclosed in Japanese Patent Application Laid-Open Publication No. 2007-215583 has a magnetic field generating unit that includes a pair of magnetic field generating parts disposed on a rotary table. The magnetic field generating device is configured to perform three-dimensional magnetic field control by combining a rotational position of the rotary table with a rotational position of the pair of magnetic field generating parts. The known technology of this kind of magnetic field generating unit may also be diverted for use as a mechanism that is capable of changing a visual field direction or spraying direction or the like of various medical instruments (the intra-abdominal camera 4, the pharmaceutical spraying apparatus 80, the image pickup apparatus 95 and the like) installed inside the abdominal cavity 101 by operation of the extracorporeal device 3 according to the present embodiment.

The invention described in each of the above embodiments is not limited to the embodiments and modifications, and may be effected by making various modifications without departing from the scope of the appended claims.

## Claims

1. A medical apparatus (1), comprising:
a medical instrument (4) that is introduced into a body cavity;
a fixing portion (42) for fixing the medical instrument (4) to a body wall inside the body cavity;
a driven posture control portion (44) connected to the medical instrument (4) such that a posture of the medical instrument (4) can be changed, and rotably connected to the fixing portion (42);
a movable portion (66) which is interposed between the medical instrument (4) and the fixing portion (42) and movably connects the medical instrument (4) to the fixing portion (42);
an extracorporeal device (3) that is installed on a body surface outside the body and has a posture control portion (22) that is rotatably provided and moves the driven posture control portion (44) in a contactless manner from outside the body so that a posture position of the medical instrument (4) can be changed relative to the fixing portion (42); and
a posture position fixing member (35) provided to the extracorporeal device (3), for fixing a rotation position of the posture control portion (22) and fixing the posture position of the medical instrument (4) relative to the fixing portion (42).

2. The medical apparatus (1) according to claim 1, wherein:
a ferromagnetic body (25, 63) is disposed in the driven posture control portion (44) and the posture control portion (22), respectively; and
a posture of the medical instrument (4) can be changed by means of mutual magnetic forces of the ferromagnetic bodies (25, 63).

3. The medical apparatus (1) according to claim 2, wherein the ferromagnetic body (25, 63) is a permanent magnet.

4. The medical apparatus (1) according to any one of claims 1 to 3, wherein the movable portion (66) is a ball joint comprising a sphere (62) and a holding portion (64) that rotatably holds the sphere (62) at a predetermined angle.

5. The medical apparatus (1) according to any one of claims 1 to 4, wherein the medical instrument (4) comprises a functional portion that is controllable from outside a body.

6. The medical apparatus (1) according to claim 5, wherein the medical instrument (4) comprises a transmitter or a receiver and controls the functional portion by means of a radio signal from outside the body.

7. The medical apparatus (1) according to any one of claims 1 to 6, wherein the medical instrument (4) is an image pickup apparatus.

## Patentansprüche

1. Medizinisches Gerät (1), das umfasst:
ein medizinisches Instrument (4), das in eine Körperhöhle eingeführt wird;
einen Fixierungsteil (42) zum Fixieren des medizinischen Instruments (4) an einer Körperwand im Inneren der Körperhöhle;
einen angetriebenen Stellungssteuerteil (44), der mit dem medizinischen Instrument (4) verbunden ist, so dass eine Stellung des medizinischen Instruments (4) geändert werden kann, und drehbar mit dem Fixierungsteil (42) verbunden ist;
einen bewegbaren Teil (66), der zwischen dem medizinischen Instrument (4) und dem Fixierungsteil (42) angeordnet ist und das medizinische Instrument (4) bewegbar mit dem Fixierungsteil (42) verbindet;
eine extrakorporale Vorrichtung (3), die auf einer Körperoberfläche außerhalb des Körpers angebracht wird und einen Stellungssteuerteil (22) aufweist, der drehbar vorgesehen ist und den angetriebenen Stellungssteuerteil (44) auf berührungslose Weise von außerhalb des Körpers bewegt, so dass eine Stellungsposition des medizinischen Instruments (4) in Bezug auf den Fixierungsteil (42) geändert werden kann; und
einen Stellungspositionsfixierungsteil (35), der zu der extrakorporalen Vorrichtung (3) vorgesehen ist, zum Fixieren einer Drehposition des Stellungssteuerteils (22) und Fixieren der Stellungsposition des medizinischen Instruments (4) in Bezug auf den Fixierungsteil.

2. Medizinisches Gerät (1) nach Anspruch 1, wobei:
ein ferromagnetischer Körper (25, 63) in dem angetriebenen Stellungssteuerteil (44) bzw. dem Stellungssteuerteil (22) angeordnet ist und
eine Stellung des medizinischen Instruments (4) mittels gegenseitiger magnetischer Kräfte der ferromagnetischen Körper (25, 63) geändert werden kann.

3. Medizinisches Gerät (1) nach Anspruch 2, wobei der ferromagnetische Körper (25, 63) ein Permanentmagnet ist.

4. Medizinisches Gerät (1) nach einem der Ansprüche 1 bis 3, wobei der bewegbare Teil (66) ein Kugelgelenk ist, das eine Sphäre (62) und einen Halteteil (64) umfasst, der die Sphäre (62) in einem vorherbestimmten Winkel drehbar hält.

5. Medizinisches Gerät (1) nach einem der Ansprüche 1 bis 4, wobei das medizinische Instrument (4) einen Funktionsteil umfasst, der von außerhalb eines Körpers steuerbar ist.

6. Medizinisches Gerät (1) nach Anspruch 5, wobei das medizinische Instrument (4) einen Sender oder einen Empfänger umfasst und den Funktionsteil mittels eines Funksignals von außerhalb des Körpers steuert.

7. Medizinisches Gerät (1) nach einem der Ansprüche 1 bis 6, wobei das medizinische Instrument (4) ein Bildaufnahmegerät ist.

## Revendications

1. Appareil médical (1), comprenant :
un instrument médical (4) qui est introduit dans une cavité corporelle ;
une partie de fixation (42) pour fixer l'instrument médical (4) à une paroi corporelle à l'intérieur de la cavité corporelle ;
une partie entraînée de commande de posture (44) reliée à l'instrument médical (4) de telle sorte qu'une posture de l'instrument médical (4) peut être changée, et reliée de manière rotative à la partie de fixation (42) ;
une partie mobile (66) qui est interposée entre l'instrument médical (4) et la partie de fixation (42) et relie de manière mobile l'instrument médicale (4) à la partie de fixation (42) ;
un dispositif extracorporel (3) qui est installé sur une surface de corps à l'extérieur du corps et a une partie de commande de posture (22) qui est disposée de manière rotative et déplace la partie entraînée de commande de posture (44) d'une manière sans contact depuis l'extérieur du corps de telle sorte qu'une position de posture de l'instrument médical (4) peut être changée par rapport à la partie de fixation (42) ; et
un élément de fixation de position de posture (35) prévu sur le dispositif extracorporel (3), pour fixer une position de rotation de la partie de commande de posture (22) et fixer la position de posture de l'instrument médical (4) par rapport à la partie de fixation (42).

2. Appareil médical (1) selon la revendication 1, dans lequel :
un corps ferromagnétique (25, 63) est disposé dans la partie entraînée de commande de posture (44) et la partie de commande de posture (22), respectivement ; et
une posture de l'instrument médical (4) peut être changée au moyen de forces magnétiques réciproques des corps ferromagnétiques (25, 63).

3. Appareil médical (1) selon la revendication 2, dans lequel le corps ferromagnétique (25, 63) est un aimant permanent.

4. Appareil médical (1) selon l'une quelconque des revendications 1 à 3, dans lequel la partie mobile (66) est un joint à rotule comprenant une sphère (62) et une partie de maintien (64) qui maintient la sphère (62) de manière rotative à un angle prédéterminé.

5. Appareil médical (1) selon l'une quelconque des revendications 1 à 4, dans lequel l'instrument médical (4) comprend une partie fonctionnelle qui est commandable depuis l'extérieur d'un corps.

6. Appareil médical (1) selon la revendication 5, dans lequel l'instrument médical (4) comprend un émetteur ou un récepteur et commande la partie fonctionnelle au moyen d'un signal radio provenant de l'extérieur du corps.

7. Appareil médical (1) selon l'une quelconque des revendications 1 à 6, dans lequel l'instrument médical (4) est un appareil de capture d'image.
